# EUROPEAN PATENT APPLICATION

(11) **EP 4 524 981 A1**
(43) Date of publication of application: **19.03.2025**
(21) Application number: 23846050.5
(22) Date of filing: 14.06.2023
(51) Int. Cl.: G16H 20/00

(54) **COMPUTER PROGRAM, INFORMATION PROCESSING DEVICE, INFORMATION PROCESSING METHOD, AND TRAINING MODEL GENERATION METHOD**

(30) Priority: 25.07.2022 JP 2022118144
(71) Applicant: Terumo Kabushiki Kaisha, Tokyo 151-0072 (JP)
(72) Inventor: SHIMADA, Naoya, Ashigarakami-gun, Kanagawa 259-0151 (JP); KIKUCHI, Hideka, Ashigarakami-gun, Kanagawa 259-0151 (JP); YAGO, Seiji, Ashigarakami-gun, Kanagawa 259-0151 (JP); MARUYAMA, Tomoji, Ashigarakami-gun, Kanagawa 259-0151 (JP); TAKEUCHI, Takanori, Tokyo 163-1450 (JP); YOKOUCHI, Atsushi, Tokyo 163-1450 (JP); FUJII, Naoko, Ashigarakami-gun, Kanagawa 259-0151 (JP)
(74) Representative: KIPA AB
(86) International application number: PCT/JP2023/022148
(87) International publication number: WO 2024/024316

(57) **Abstract**

A computer program, an information processing device, an information processing method, and a learning model generation method that provide an appropriate coping method are provided.

The computer program causes a computer to execute processing including acquiring pain related information regarding pain of a patient, in a case where the pain related information is input, inputting the acquired pain related information into a learning model that outputs a pain evaluation result and acquiring the pain evaluation result of the patient, generating coping information for coping with the pain based on the acquired pain evaluation result, and outputting the generated coping information.

## Description

### Technical Field

The present invention relates to a computer program, an information processing device, an information processing method, and a learning model generation method.

### Background Art

Patent Literature 1 discloses a medical information management system that includes a plurality of medical department servers that provides medical information stored in a database of a plurality of medical departments in a medical institution and a specific disease treatment integrated server that acquires specific disease medical information from the medical department server and provides the integrated specific disease medical information from an integrated database, in which a treatment progressing status can be grasped with high immediacy by making it possible to refer to medical information of specific diseases such as cancer in all treatment methods combining a surgery, radiation therapy, chemotherapy, and palliative care and registered related information of the specific diseases **in** a wide range.

### Citation List

### Patent Literature

Patent Literature 1: JP 2010-3135 A

### Summary of Invention

### Technical Problem

Pain caused by diseases such as cancer is directly linked to quality of life (QOL) of a patient. It is often difficult for a patient to accurately tell pain to a doctor, there is no general methodology for deriving an appropriate pain treatment because there are various symptoms of the patients, and it is difficult for the doctor to determine an appropriate pain treatment. Furthermore, although the pain treatment is performed at the time of examination by the doctor, the patient needs to withstand the pain even if the symptoms change **in** a period other than the time of examination.

The present invention has been made **in** view of the above circumstances, and an object of the present invention is to provide a computer program, an information processing device, an information processing method, and a learning model generation method that can provide an appropriate coping method. Specifically, an appropriate coping method necessary at the time when a patient feels pain is provided at an appropriate timing, based on information at the time when the patient feels pain, including a time other than the time of medical care.

### Solution to Problem

(1) A computer program according to the present invention causes a computer to execute processing including acquiring pain related information regarding pain of a patient, in a case where the pain related information is input, inputting the acquired pain related information into a learning model that outputs a pain evaluation result and acquiring the pain evaluation result of the patient, generating coping information for coping with the pain based on the acquired pain evaluation result, and outputting the generated coping information.
   Here, in an embodiment of the present invention,
(2) the computer program according to (1) in which the pain related information preferably includes at least one of an evaluation index used to evaluate pain, vital data, medication information, and a physical condition.
(3) The computer program according to (1) or (2), preferably causes the computer to execute processing further including acquiring medical information regarding medical care of the patient, and in a case where the medical information is further input, inputting the acquired medical information into the learning model that outputs the pain evaluation result and acquiring the pain evaluation result of the patient.
(4) The computer program according to any one of (1) to (3), in which the medical information preferably includes at least one of diagnosis information, examination information, treatment information, prescription information, and disease prognosis prediction information.
(5) The computer program according to any one of (1) to (4), preferably causes the computer to execute processing further including acquiring literature information regarding a pain treatment, and in a case where the literature information regarding the pain treatment is further input, inputting the acquired literature information into the learning model that outputs the pain evaluation result and acquiring the pain evaluation result of the patient.
(6) The computer program according to any one of (1) to (5), in which the pain evaluation result preferably includes a pain progress of the patient from the past to the future.
(7) The computer program according to any one of (1) to (6), preferably causes the computer to execute processing further including receiving a situation of the patient as a result of performing the coping information and recommending examination of a doctor according to the received situation.
(8) The computer program according to any one of (1) to (7), preferably causes the computer to execute processing further including receiving a situation of the pain of the patient as a result of performing a pain treatment based on the coping information, correcting the pain evaluation result based on the received situation of the pain, and relearning the learning model using the corrected pain evaluation result.
(9) The computer program according to any one of (1) to (8), preferably causes the computer to execute processing further including receiving an operation for correcting the coping information generated based on the pain evaluation result of the patient from a doctor terminal device.
(10) The computer program according to any one of (1) to (9), preferably causes the computer to execute processing further including outputting a question generated based on the pain evaluation result of the patient to a patient terminal device and outputting coping information to the patient terminal device according to an answer of the patient to the question.
(11) The computer program according to any one of (1) to (10), preferably causes the computer to execute processing further including receiving consultation from the patient and outputting the coping information to the patient terminal device according to the received consultation.
(12) The computer program according to any one of (1) to (11), preferably causes the computer to execute processing further including receiving arrangement of medicine or contact to a doctor, together with the coping information.
(13) The computer program according to any one of (1) to (12), preferably causes the computer to execute processing including controlling automatic administration of medicine in a medicine injection pump based on the pain evaluation result of the patient.
(14) An information processing device according to the present invention includes a first acquisition unit that acquires pain related information regarding pain of a patient, a second acquisition unit that inputs the acquired pain related information into a learning model that outputs a pain evaluation result, in a case of inputting the pain related information and acquires the pain evaluation result of the patient, a generation unit that generates coping information for coping with the pain based on the acquired pain evaluation result, and an output unit that outputs the generated coping information.
(15) An information processing method according to the present invention includes acquiring pain related information regarding pain of a patient, inputting the acquired pain related information into a learning model that outputs a pain evaluation result, in a case where the pain related information is input, and acquiring the pain evaluation result of the patient, generating coping information for coping with the pain based on the acquired pain evaluation result, and outputting the generated coping information.
(16) A learning model generation method includes acquiring first training data including pain related information regarding pain of a plurality of patients and pain evaluation results of the plurality of patients and generating a learning model so as to output the pain evaluation result in a case where the pain related information is input, based on the acquired first training data.
(17) The learning model generation method according to (16), preferably acquires second training data further including medical information of the plurality of patients, literature information regarding a pain treatment, and the pain evaluation results of the plurality of patients and generates the learning model so as to output the pain evaluation result in a case where the medical information and the literature information regarding the pain treatment are input, based on the acquired second training data.

### Advantageous Effects of Invention

According to the present invention, it is possible to provide an appropriate coping method.

### Brief Description of Drawings

Fig. 1 is a diagram illustrating an example of a configuration of a pain treatment assistance system according to the present embodiment.
Fig. 2 is a diagram illustrating an example of a configuration of a pain treatment assistance device.
Fig. 3 is a diagram illustrating information collection by the pain treatment assistance device.
Fig. 4A is a diagram illustrating an example of information in a database.
Fig. 4B is a diagram illustrating an example of the information in the database.
Fig. 4C is a diagram illustrating an example of the information in the database.
Fig. 5 is a diagram illustrating a first example of processing for generating coping information by the pain treatment assistance device.
Fig. 6 is a diagram illustrating a second example of the processing for generating the coping information by the pain treatment assistance device.
Fig. 7 is a diagram illustrating a third example of the processing for generating the coping information by the pain treatment assistance device.
Fig. 8 is a diagram illustrating an example of a learning model generation method.
Fig. 9 is a diagram illustrating a first example of the coping information.
Fig. 10 is a diagram illustrating a second example of the coping information.
Fig. 11 is a diagram illustrating a third example of the coping information.
Fig. 12 is a diagram illustrating a fourth example of the coping information.
Fig. 13 is a diagram illustrating a fifth example of the coping information.
Fig. 14 is a diagram illustrating a sixth example of the coping information.
Fig. 15 is a diagram illustrating a seventh example of the coping information.
Fig. 16 is a diagram illustrating an eighth example of the coping information.
Fig. 17 is a diagram illustrating a ninth example of the coping information.
Fig. 18 is a diagram illustrating a tenth example of the coping information.
Fig. 19 is a diagram illustrating an eleventh example of the coping information.
Fig. 20 is a diagram illustrating a twelfth example of the coping information.
Fig. 21 is a diagram illustrating a thirteenth example of the coping information.
Fig. 22 is a diagram illustrating an example of a coping information correction screen.
Fig. 23 is a diagram illustrating an example of a processing procedure by the pain treatment assistance device.
Fig. 24 is a diagram illustrating an example of a learning model generation procedure.

### Description of Embodiments

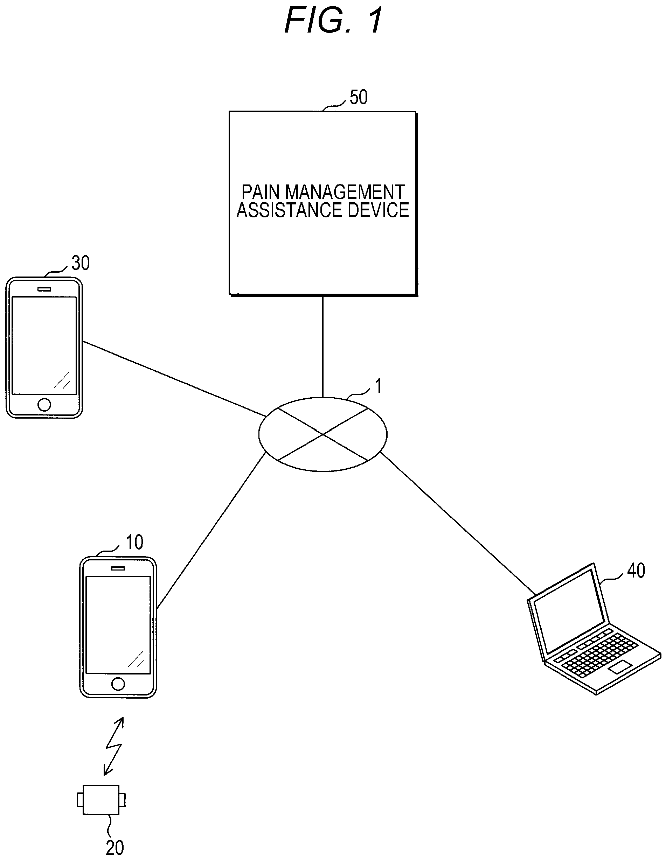

Hereinafter, an embodiment of the present invention will be described. Fig. 1 is a diagram illustrating an example of a configuration of a pain treatment assistance system according to the present embodiment. The pain treatment assistance system as an information processing system includes a pain treatment assistance device 50 as an information processing device. The pain treatment assistance system may include a patient terminal device 10, a device 20, an assistant terminal device 30, and a doctor terminal device 40. The patient terminal device 10, the assistant terminal device 30, and the doctor terminal device 40 are connected to the pain treatment assistance device 50 via a communication network 1. The device 20 may have a configuration that is wirelessly or wiredly connected to the patient terminal device 10 or may have a configuration that is connected to the pain treatment assistance device 50 via the communication network 1. Note that the device 20 is not essential, and for example, a configuration may include only input from the patient terminal device 10.

The patient terminal device 10 is a terminal device carried or held by a patient, and the assistant terminal device 30 is a terminal device carried or held by an assistant such as a family member or a caregiver of the patient. The patient terminal device 10 and the assistant terminal device 30 can include a smartphone, a tablet, a personal computer, or the like including a display panel, an operation panel, a microphone, a speaker, or the like. In the patient terminal device 10 and the assistant terminal device 30, an app (application) for using the pain treatment assistance system is introduced. For example, a patient who is suffering from cancer or the like and periodically visits a medical institution while being treated at home is assumed. However, the patient is not limited to such a patient. Note that the app may be a Web app or a downloaded app.

The device 20 is a measuring instrument that measures vital data of the patient and may be a wearable terminal or a stationary type. The device 20 can measure the vital data such as a body temperature, a heart rate, brain waves, or electrodermal activity (EDA). Note that the vital data is not limited to these, and may include any data assumed to be related to pain caused by the patient's disease. For example, a blood pressure, a pulse pressure, pulse waves, a blood sugar level, a weight, the number of steps, an activity amount, or the like may be included in the vital data.

The doctor terminal device 40 is a terminal device used by a doctor in a medical institution or the like. The doctor terminal device 40 can include a personal computer, a tablet, or the like including a display panel, an operation panel, or the like. A medical worker such as a public health nurse or a nurse may use the doctor terminal device 40 under guidance of the doctor. An application (for example, Web app or the like) for using the pain treatment assistance system is introduced in the doctor terminal device 40.

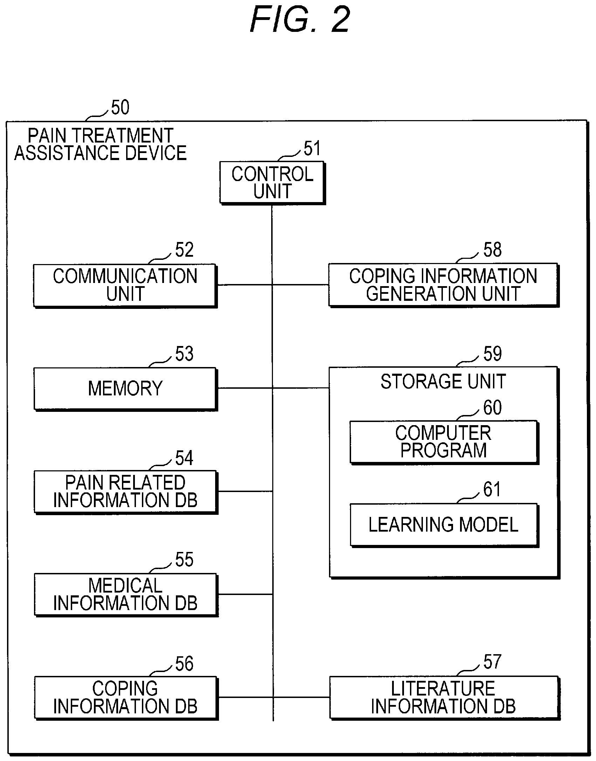

Fig. 2 is a diagram illustrating an example of a configuration of the pain treatment assistance device 50. The pain treatment assistance device 50 includes a control unit 51 that controls an entire device, a communication unit 52, a memory 53, a pain related information DB 54, a medical information DB 55, a coping information DB 56, a literature information DB 57, a coping information generation unit (generation unit) 58, and a storage unit 59. The pain treatment assistance device 50 may distribute processing functions and include a plurality of devices. A database such as the pain related information DB 54, the medical information DB 55, the coping information DB 56, or the literature information DB 57 may be built in the pain treatment assistance device 50 or may be incorporated into an external data server. Furthermore, the storage unit 59 may be incorporated into an external data server.

The storage unit 59 can include, for example, a hard disk, a semiconductor memory, or the like, and stores a computer program 60 (program product), a learning model 61, and necessary information. The computer program 60 may be downloaded from an external device via the communication unit 52 and stored in the storage unit 59. Furthermore, the computer program 60 recorded in a recording medium (for example, optically readable disk storage medium such as CD-ROM) may be read by a recording medium reading unit and stored in the storage unit 59, or the computer program 60 may be read by the recording medium reading unit and developed in the memory 53.

The control unit 51 may be configured by incorporating a required number of central processing units (CPUs), micro-processing units (MPUs), graphics processing units (GPUs), or the like. The control unit 51 can execute processing defined by the computer program 60. That is, the processing executed by the control unit 51 corresponds to processing executed in accordance with the computer program 60. The control unit 51 can execute functions of a coping information generation unit 58, by executing the computer program 60. The coping information generation unit 58 may include hardware or software or may be implemented by a combination of hardware and software. The control unit 51 can execute processing using the learning model 61. Note that the control unit 51 has functions as a first acquisition unit, a second acquisition unit, a generation unit, and an output unit.

The communication unit 52 includes a communication module and can exchange necessary information with the patient terminal device 10 and the doctor terminal device 40 via the communication network 1. Furthermore, the communication unit 52 may exchange necessary information with the device 20.

The memory 53 may include a semiconductor memory, such as a static random access memory (SRAM), a dynamic random access memory (DRAM), or a flash memory. Loading the computer program 60 into the memory 53 enables the control unit 51 to execute the computer program 60.

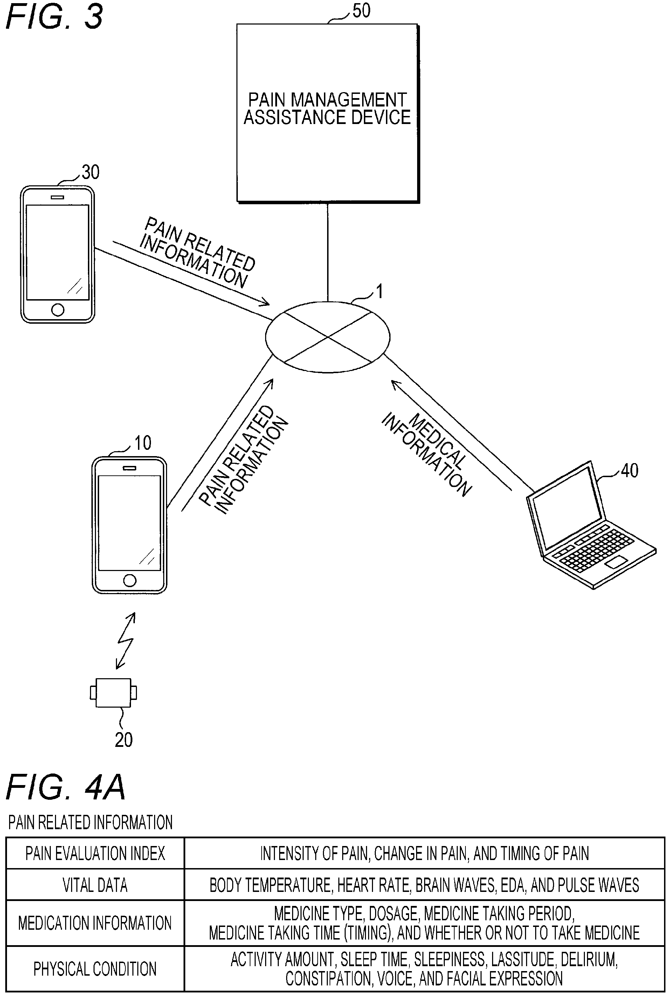

Fig. 3 is a diagram illustrating information collection by the pain treatment assistance device 50. The pain treatment assistance device 50 acquires pain related information related to pain caused by the patient's disease from the patient terminal device 10 and the device 20 via the communication unit 52. The pain related information is repeatedly transmitted from the patient terminal device 10 and the device 20 as appropriate. For example, the patient may transmit the pain related information once in a period from a previous hospital visit to a next hospital visit or may transmit the pain related information a plurality of times according to a length of the period. The acquired pain related information is classified for each patient and for each acquired time point and stored in the pain related information DB 54.

Furthermore, the pain treatment assistance device 50 acquires medical information related to medical care of the patient from the doctor terminal device 40 via the communication unit 52. The acquired medical information is classified for each patient and for each medical care time point and stored in the medical information DB 55.

Figs. 4A to 4C are diagrams illustrating an example of information in the database. Fig. 4A illustrates an example of information in the pain related information DB 54. The pain related information can be classified into a pain evaluation index (evaluation index used to evaluate pain), vital data, medication information, and a physical condition. The pain related information transmitted from the patient terminal device 10 and the device 20 includes at least one of the pain evaluation index, the vital data, the medication information, and the physical condition. Note that the classification is an example and is not limited to the example in Fig. 4A.

The pain evaluation index includes an intensity of pain, a change in the pain, a timing of the pain, or the like. The intensity of the pain can be expressed in stages, for example, as one to 10 or one to five. The change in the pain can be expressed, for example, as gradually increasing pain, gradually decreasing pain, persisting strong pain, persisting weak pain, or the like. The timing of the pain indicates when pain occurs, and can be expressed, for example, as at the time of waking up, daytime, nighttime, at the time of sitting, at the time of standing up, or the like. The pain evaluation index can be transmitted from the patient terminal device 10 to the pain treatment assistance device 50, for example, when the patient activates the app of the patient terminal device 10 and answers a question regarding the intensity of the pain, the change in the pain, the timing of the pain, or the like.

The vital data includes the body temperature, the heart rate, the brain waves, the EDA, the pulse waves, or the like that changes in response to the pain. Note that the blood pressure, the pulse pressure, the blood sugar level, the weight, the number of steps, the activity amount, or the like may be included. The vital data can be acquired using the device 20.

The medication information includes a medicine type, a dosage, a medicine taking period, a medicine taking time (timing), whether or not to take the medicine, or the like. The medication information may include not only a medicine that is currently taken but also a medicine that has been taken in the past.

The physical condition includes an activity amount, a sleep time, sleepiness, lassitude, delirium, constipation, voice, facial expression, or the like. It is possible to include an item that can evaluate whether or not a disorder occurs due to the pain, the medicine, medical conditions, or the like.

The pain related information includes at least one of the pain evaluation index (evaluation index used to evaluate pain), the vital data, the medication information, and the physical condition.

By acquiring the pain related information as described above from the patient terminal device 10 and the device 20, the patient can accurately tell pain and symptoms that the patient feels at a necessary timing and a necessary frequency, in a period from a previous examination to a next examination.

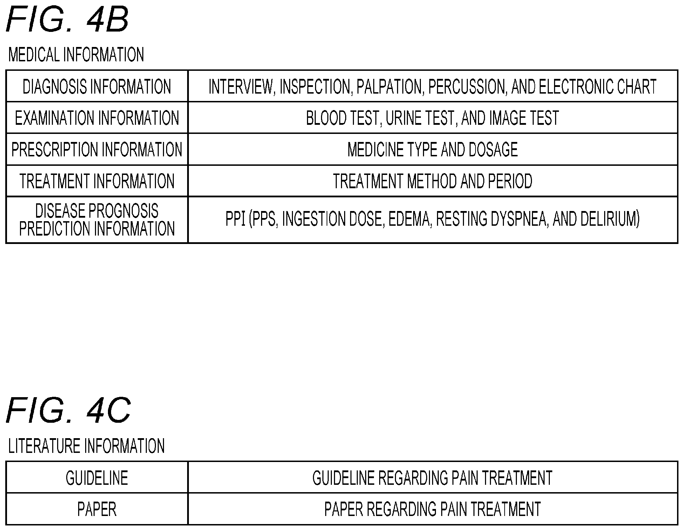

Fig. 4B illustrates an example of information in the medical information DB 55. The medical information can be classified into diagnosis information, examination information, prescription information, treatment information, and disease prognosis prediction information. The medical information transmitted from the doctor terminal device 40 includes at least one of the diagnosis information, the examination information, the prescription information, the treatment information, and the disease prognosis prediction information. Note that the classification is an example and is not limited to the example in Fig. 4B.

The diagnosis information includes findings by interview, inspection, palpation, or percussion at the time of examination, an electronic chart, or the like. The examination information includes examination result data of a blood test, a urine test, an image test, or the like. The prescription information includes a medicine type and a dosage prescribed by a doctor. Furthermore, the medicine taking period may be included. The treatment information includes a treatment method and a treatment period. Note that not only a literal treatment but also medicine prescription are included in a pain treatment. The disease prognosis prediction information includes a palliative prognostic index (PPI). The PPI is a representative index as an index used to predict a short-term life prognosis (in weeks), and a score obtained by adding scores of a palliative performance scale (PPS), an ingestion dose, an edema, resting dyspnea, and a delirium. As the score increases, predicted prognosis is shorter.

The medical information includes at least one of the diagnosis information, the examination information, the treatment information, the prescription information, and the disease prognosis prediction information.

Fig. 4C illustrates an example of information in the literature information DB 57. The literature information can be classified into a guideline and a paper. The guideline is a guideline regarding the pain treatment, and includes, for example, a guideline indicating what type of pain treatment is recommended for a symptom and pain of the patient. The paper is a paper regarding the pain treatment and includes, for example, objective information indicating what type of pain treatment is effective for the symptom and the pain of the patient. The literature information DB 57 is appropriately updated, and the latest information is recorded.

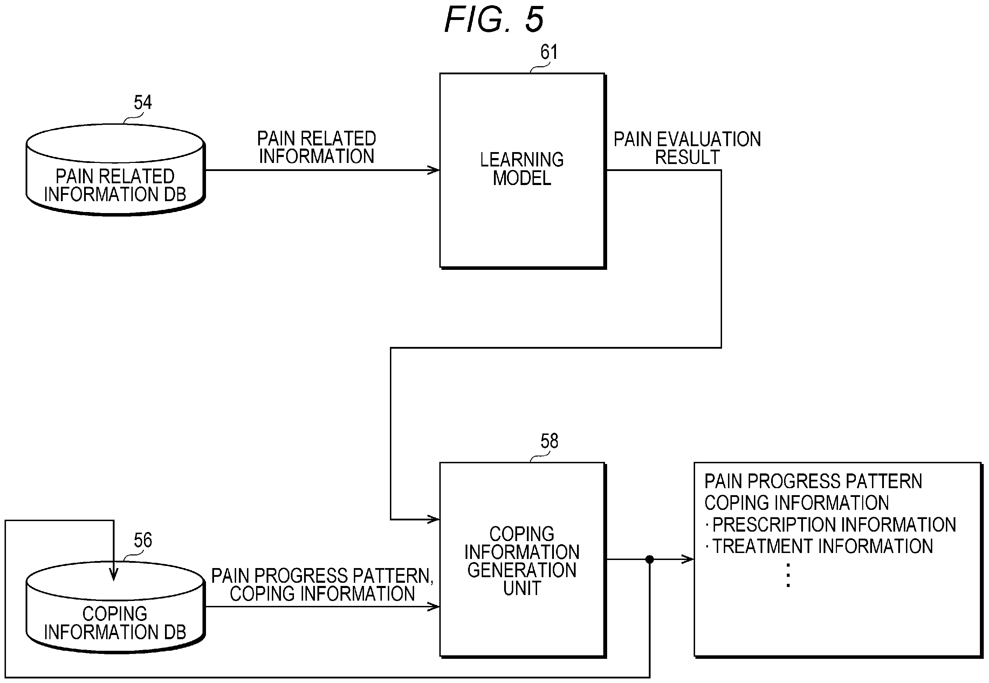

Fig. 5 is a diagram illustrating a first example of processing for generating coping information by the pain treatment assistance device 50. The control unit 51 reads pain related information of a specific patient from the pain related information DB 54 and inputs the read pain related information into the learning model 61. The pain related information of the specific patient is time-series data at a single or a plurality of time points. For example, not only current pain related information of the specific patient but also pain related information at a past time point going back from the current time point may be included. The control unit 51 can generate a pain related information vector by performing a vectorization operation for generating a primary vector for the pain related information of the patient and input the generated pain related information vector into the learning model 61. Note that the pain related information vector may be generated for each of the plurality of time points. Note that, although not illustrated, the coping information in the coping information DB 56 and a pain progress pattern may be input into the learning model 61.

The learning model 61 is generated (learned) so as to output a pain evaluation result, in a case where the pain related information is input. The pain related information is time-series data and includes, for example, pain related information within a period of about one month, three months, six months, or 12 months in the past going back from the present. The pain evaluation result includes a progress of a degree (degree) of pain of the patient from the past to the future. The past period can be set to, for example, one month, three months, six months, 12 months, or the like. Furthermore, the period from the current point to the future can be set to one month, three months, six months, 12 months, or the like. The length of the period may be determined in advance or can be set to a desired period. The learning model 61 may, for example, output pain evaluation results from past three months to future three months, based on pain related information in the past three months or may output a pain evaluation result from past three months to future three months, based on pain related information in past six months. Note that these periods are examples, and the present invention is not limited to these. The degree (degree) of the pain can be expressed, for example, in several stages such as 10 stages from one to 10 or five stages from one to five. It can be assumed that the pain of the patient be stronger as a numerical value increases.

The learning model 61 can use, for example, a support vector machine (SVM), a decision tree, a random forest, an adaboost, a neural network (for example, recurrent neural network (RNN), long short term memory (LSTM), transformer, or the like), or the like.

The control unit 51 reads the coping information (for example, including prescription information, treatment information, or the like) recorded in the coping information DB 56 and the pain progress pattern. The control unit 51 inputs the read coping information and pain progress pattern and the pain evaluation result acquired from the learning model 61 into the coping information generation unit 58.

The coping information generation unit 58 generates the pain progress pattern of the patient, based on the pain evaluation result, the pain progress pattern, and the coping information that have been input. Specifically, it is possible to complement the pain evaluation result at a time point between the plurality of times points, based on the pain evaluation results at the plurality of time points and to generate the pain progress pattern. Furthermore, the coping information generation unit 58 generates the coping information for the patient, based on the pain evaluation result and the coping information that have been input. The coping information generation unit 58 stores the generated pain progress pattern and coping information in the coping information DB 56. The coping information includes, for example, the prescription information, the treatment information, or the like. The prescription information can be determined by the doctor in advance as necessary. For example, a dosage range may be determined in advance.

It is sufficient that the pain progress pattern be information indicating a temporal transition of a degree of pain of the patient from the past to the future. The pain progress pattern can be displayed by an appropriate method such as graph display or tabular display. The prescription information is information provided to the patient by the pain treatment assistance device 50 instead of the doctor and information regarding the prescription of medicines, selected according to a situation of the pain of the patient. The treatment information is information provided to the patient by the pain treatment assistance device 50 instead of the doctor and information regarding the treatment method, selected according to the situation of the pain of the patient.

The coping information generation unit 58 may generate the coping information on a rule basis, for example, using a table indicating a correspondence relationship between the pain evaluation result and the coping information. The table may indicate the correspondence relationship between the pain evaluation result and the coping information. For example, in a case where the pain evaluation result (progress of degree (degree) of pain of patient from past to future) increases, prescription information (coping information) for changing the medicine or medication by a pump (coping information) can be associated. Furthermore, in a case where the pain evaluation result fluctuates, the examination (coping information) by the doctor can be associated. Furthermore, in a case where the pain evaluation result does not increase and is stable, coping information for maintaining a current coping method can be associated.

Furthermore, the coping information generation unit 58 can be configured by a model using a machine-learned algorithm and can use, for example, a support vector machine (SVM), a decision tree, a random forest, an adaboost, a neural network, or the like. In a case where the coping information generation unit 58 is configured by the model using the machine-learned algorithm, processing by the model is as follows, for example. The model is generated to output the coping information in a case where the pain evaluation result is input. The control unit 51 can input the pain evaluation result of the patient into the model and acquire the coping information output by the model. The control unit 51 can output the acquired coping information. Training data used for machine learning can be collected, for example, as follows. It is sufficient to collect data, in which how the pain of the patient progresses is associated with what type of coping method is adopted by the doctor, using the electronic charts or the like of the plurality of patients.

As described above, the control unit 51 can acquire the pain related information regarding the pain of the patient, input the acquired pain related information into the learning model 61 that outputs the pain evaluation result in a case where the pain related information is input and acquire the pain evaluation result of the patient, generate the coping information used to cope with the pain based on the acquired pain evaluation result, and output the generated coping information.

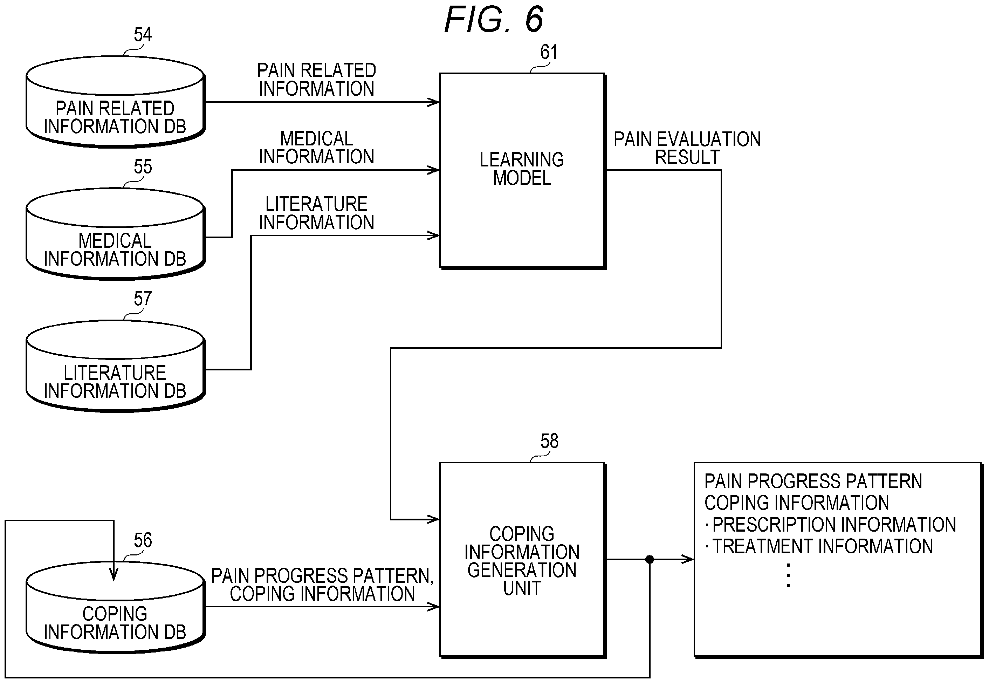

Fig. 6 is a diagram illustrating a second example of the processing for generating the coping information by the pain treatment assistance device 50. A difference from the first example illustrated in Fig. 5 is a point that the medical information and the literature information, in addition to the pain related information, are input into the learning model 61. The control unit 51 reads medical information of a specific patient from the medical information DB 55 and inputs the read medical information into the learning model 61. The medical information of the specific patient is time-series data at a single or a plurality of time points. For example, not only current medical information of the specific patient but also medical information at a past time point going back from the current time point may be included. The control unit 51 can perform the vectorization operation for generating the primary vector for the medical information, generate a medical information vector, and input the generated medical information vector into the learning model 61. Note that the medical information vector may be generated for each of the plurality of time points. The learning model 61 may, for example, output a pain evaluation result from past three months to one future month, based on the pain related information in the past three months and the medical information or may output a pain evaluation result from past six months to future three months, based on the pain related information in past six months and the medical information. Note that, although not illustrated, the coping information in the coping information DB 56 and the pain progress pattern may be input into the learning model 61.

Furthermore, the control unit 51 reads the literature information from the literature information DB 57 and inputs the read literature information into the learning model 61. The control unit 51 can perform the vectorization operation for generating the primary vector for the literature information, generate a literature information vector, and input the generated literature information vector into the learning model 61. It is sufficient that the literature information include, for example, information necessary for associating a type of history information such as a symptom history of the patient, a history of the vital data, an examination history, a medication history, or a treatment history, with how the situation of the pain of the patient has changed from the past to the future. For example, if there is a paper indicating how pain changes when a specific medicine is taken, the paper can be used as the literature information. Furthermore, if there is a guideline indicating how pain changes when a certain specific treatment is performed, the guideline can be used as the literature information. In particular, by compensating shortage of the pain related information and the medical information with the literature information, in a case where the pain related information and the medical information of the patient are insufficient (for example, in a case where past data is small), the learning model 61 can output the pain evaluation result of the patient. It is expected that accuracy of an index output by the learning model 61 is improved by increasing the number of types of information to be input into the learning model 61. Note that, although not illustrated, the coping information in the coping information DB 56 and the pain progress pattern may be input into the learning model 61.

As described above, in a case of acquiring the medical information regarding the medical care of the patient and further inputting the medical information, the control unit 51 may input the acquired medical information into the learning model 61 that outputs the pain evaluation result and acquire the pain evaluation result of the patient.

Furthermore, in a case of acquiring the literature information regarding the pain treatment and further inputting the literature information regarding the pain treatment, the control unit 51 may input the acquired literature information into the learning model 61 that outputs the pain evaluation result and acquire the pain evaluation result of the patient.

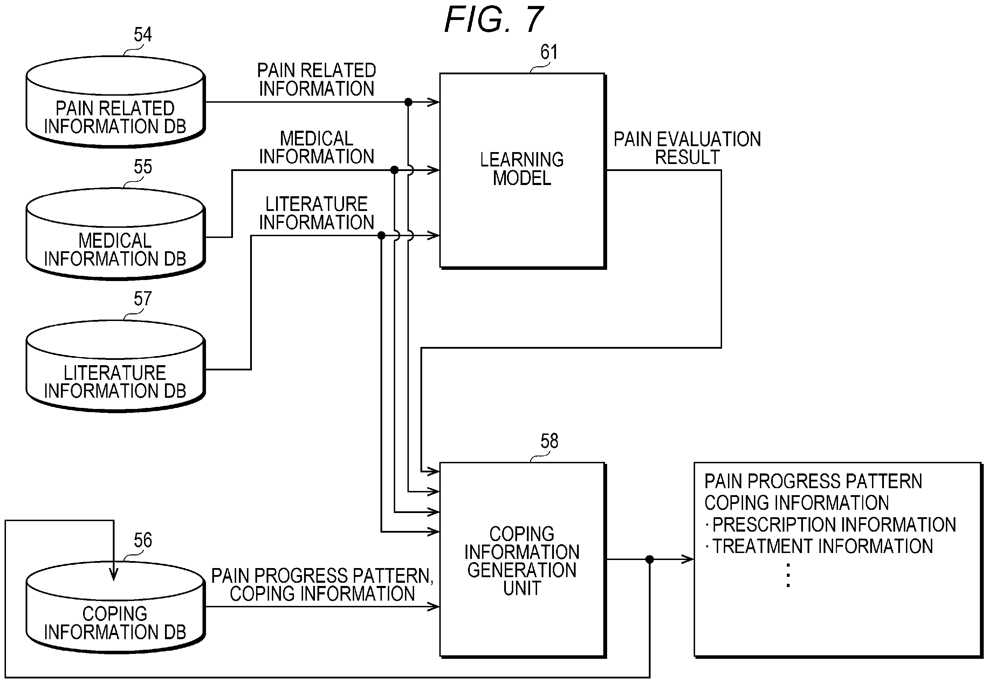

Fig. 7 is a diagram illustrating a third example of the processing for generating the coping information by the pain treatment assistance device 50. A difference from the second example illustrated in Fig. 6 is a point that the pain related information, the medical information, and the literature information of the patient, in addition to the pain evaluation result, are input into the coping information generation unit 58.

The coping information generation unit 58 may generate the coping information on a rule basis, for example, using a table indicating a correspondence relationship between the pain evaluation result, the pain related information, the medical information, and the literature information of the patient and the coping information. The table may indicate the correspondence relationship between the pain evaluation result, the pain related information, the medical information, and the literature information of the patient, and the coping information. For example, for example, in a case where the pain evaluation result (progress of degree (degree) of pain of patient from past to future) increases and the pain based on the pain related information of the patient is relatively strong and continues for a relatively long time, it is possible to associate coping information so as to stop taking the medicine and contact the doctor. Furthermore, in a case where the pain evaluation result is stable without increasing and examination result data has no problem, it is possible to associate the coping information so as to maintain the current coping method.

Furthermore, as in the first and second examples, the coping information generation unit 58 can be configured by the model using the machine-learned algorithm and can use, for example, a support vector machine (SVM), a decision tree, a random forest, an adaboost, a neural network, or the like.

In a case where the coping information generation unit 58 is configured by the model using the machine-learned algorithm, processing by the model is as follows, for example. The model is generated to output the coping information in a case where the pain evaluation result, and the pain related information, the medical information, and the literature information of the patient are input. The control unit 51 can input the pain evaluation result of the patient and the pain related information, the medical information, and the literature information of the patient into the model and acquire the coping information output by the model. The control unit 51 can output the acquired pain progress pattern and coping information. The training data used for machine learning can be collected, for example, as follows. It is sufficient to collect data in which how the pain of the patient progresses and what type of the pain related information and the medical information the patient has are associated with what type of coping method is adopted by the doctor, using the electronic charts or the like of the plurality of patients. At that time, the literature information may be referred.

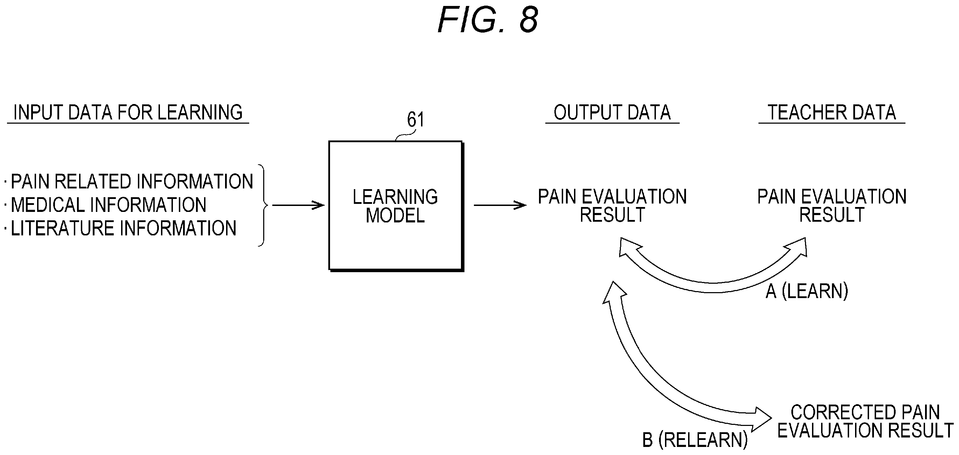

Fig. 8 is a diagram illustrating an example of a method for generating the learning model 61. Hereinafter, it is assumed that the pain treatment assistance device 50 generate the learning model 61. However, a learning processing device other than the pain treatment assistance device 50 may generate the learning model 61, and the pain treatment assistance device 50 may acquire the generated learning model 61. The control unit 51 acquires training data (second training data) including the pain related information regarding the pain of the plurality of patients, the medical information, the literature information regarding the pain treatment, and the pain evaluation results of the plurality of patients. These pieces of training data include disease progress information and pain change information of the plurality of patients. The pain related information, the medical information, and the pain evaluation result are time-series data and include, for example, data within a period of about one month, three months, six months, or 12 months.

It is sufficient that the literature information include, for example, information necessary for associating the type of history information such as the symptom history of the patient, the history of the vital data, the examination history, the medication history, or the treatment history, with how the pain of the patient has changed. For example, if there is a paper indicating how pain changes in a case where a specific medicine is taken or in a case where a specific treatment is performed, the paper can be used as the literature information. Furthermore, if there is a guideline indicating how pain changes when a certain specific treatment is performed, the guideline can be used as the literature information. The control unit 51 can generate the learning model 61 so as to output the pain evaluation result in a case where the pain related information, the medical information, and the literature information regarding the pain treatment are input, based on the acquired training data. For example, the learning model 61 can set pain related information, medical information, and literature information for first three months of six months as input data for learning, using training data including pain related information, medical information, and pain evaluation results for six months and can be learned using the pain evaluation results for the six months as teacher data. In this case, the learning model 61 can output the pain evaluation results for past three months to future three months, based on the literature information, the pain related information for the past three months, and the medical information. At the time when the learning model 61 is generated, it is sufficient to adjust a parameter of the learning model 61 so that the pain evaluation result output from the learning model 61 approaches the pain evaluation result that is the teacher data included in the training data (refer to reference A).

Note that, in the example in Fig. 8, the pain related information, the medical information, and the literature information are included in the input data for learning. However, for example, it is not necessary to include at least one of the medical information and the literature information in the input data for learning. For example, in a case of acquiring training data including the pain related information regarding the pain of the plurality of patients and the pain evaluation results of the plurality of patients (first training data) and inputting the pain related information based on the acquired training data, the control unit 51 may generate the learning model 61 so as to output the pain evaluation result. However, it can be expected that inference accuracy of the learning model 61 is improved if at least one of the medical information and the literature information is included in the input data for learning.

The control unit 51 can relearn the learning model 61. That is, in a case of acquiring the training data including the corrected pain evaluation result and inputting the pain related information, the medical information, and the literature information regarding the pain treatment based on the acquired training data, it is sufficient for the control unit 51 to readjust the parameter of the learning model 61 so that the pain evaluation result output by the learning model 61 approaches the corrected pain evaluation result that is the teacher data (refer to reference B).

It is sufficient to correct the pain evaluation result, for example, as follows. It is sufficient for the control unit 51 to receive feedback of the situation of the pain of the patient, as a result of performing the pain treatment based on the coping information on the patient and to correct the pain evaluation result according to the received situation of the pain.

Next, a specific example of the coping information will be described. The control unit 51 can output the coping information to the patient terminal device 10. The coping information is displayed on the display panel of the patient terminal device 10, and the patient can perform an input operation or a selection operation according to displayed content.

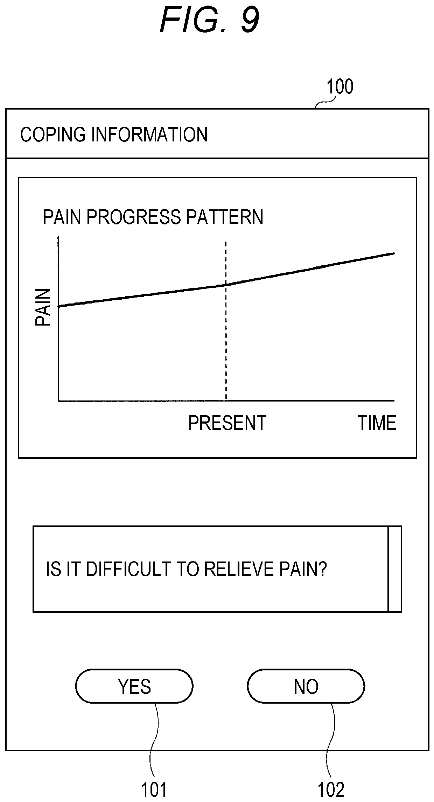

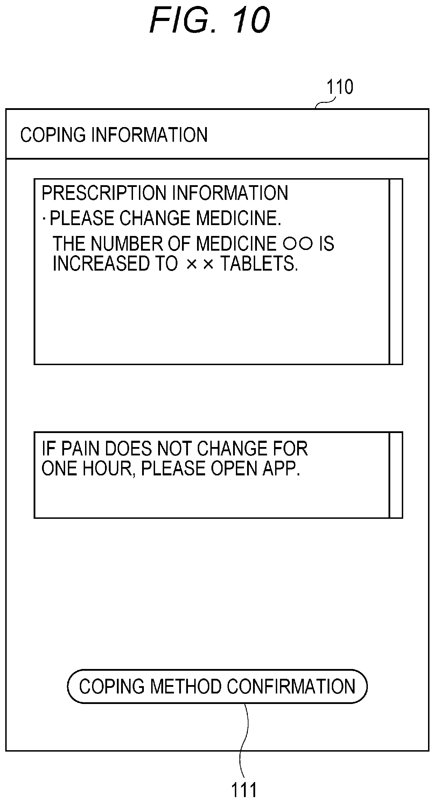

Fig. 9 is a diagram illustrating a first example of coping information 100. In the coping information 100, the pain progress pattern of the patient is displayed. In the example in Fig. 9, the pain progress pattern is displayed as a line graph, and a transition of the pain of the patient from the past to the future can be seen. A question based on the pain progress pattern is displayed for the patient as if the doctor asks a question (interview) for the patient. The question is, for example, "Is it difficult to relieve pain?". The patient can answer to the question by operating a "Yes" icon 101 or a "No" icon 102. As a result, the patient can obtain an environment similar to an environment as if the patient is examined by the doctor. When the patient operates the "Yes" icon 101, coping information illustrated in Fig. 10 to be described later is displayed.

Fig. 10 is a diagram illustrating a second example of coping information 110. The coping information 110 includes the prescription information. In the example in Fig. 10, "Please change medicine. The number of medicine OO is increased to XX tablets." is displayed as the prescription information. Furthermore, as an advice of the doctor to the patient, "If pain does not change for one hour, please open app." is displayed, and the patient can obtain an environment as if the patient talks to the doctor, by opening the app. Note that, regarding the medicine change, the doctor may determine a change option at the time of interview. When the patient operates a "coping method confirmation" icon 111, a coping method is displayed. The prescription information as illustrated in Fig. 10 can be stored in the storage unit 59 in advance as a table associated with a plurality of pain progress patterns. For example, pieces of prescription information R1, R2, R3,... are associated with pain progress patterns P1, P2, P3,.... The control unit 51 can select a pain progress pattern closest to the pain progress pattern of the patient as illustrated in Fig. 9 from the table and specify prescription information associated with the selected pain progress pattern.

As described above, the control unit 51 can output the change in the coping information to the patient terminal device 10 based on the pain evaluation result.

Furthermore, the control unit 51 can output a question generated based on the pain evaluation result of the patient to the patient terminal device 10 and output coping information to the patient terminal device 10 according to an answer of the patient to the question. As a result, the patient can obtain a coping method according to the situation of the pain from the past to the future including the present, in a period other than the time of examination by the doctor, and it is possible to improve quality of life (QOL) of the patient. In this way, an appropriate coping method can be provided.

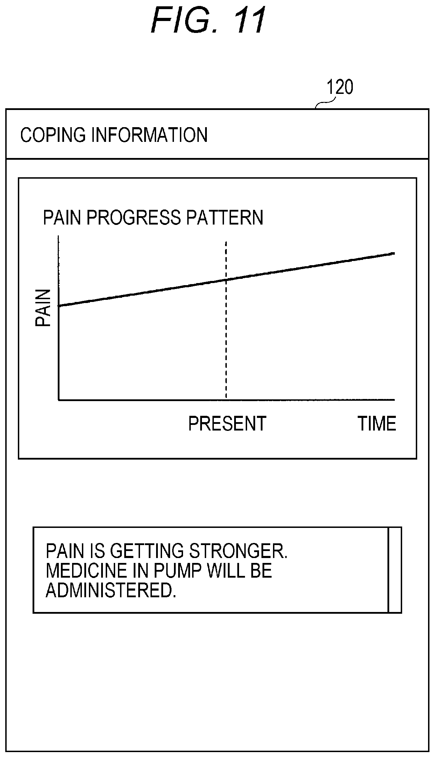

Fig. 11 is a diagram illustrating a third example of coping information 120. In the coping information 120, the pain progress pattern of the patient is displayed. In the example in Fig. 11, as in the example in Fig. 9, the pain progress pattern is displayed as a line graph, and the transition of the pain of the patient from the past to the future can be seen. Analysis of the pain and prescription based on the pain progress pattern are displayed. For example, "Pain is getting stronger. Medicine in pump will be administered". As a result, it is possible to determine the pain of the patient and automatically administer the medicine in the pump. Furthermore, an instruction may be transmitted to the medical worker, the caregiver, or the like so as to activate the pump at a remote place such as home. As a result, it is not necessary to remotely operate the pump.

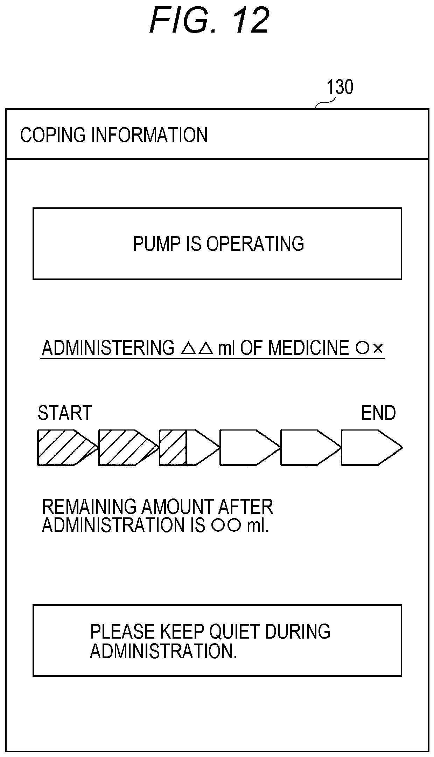

Fig. 12 is a diagram illustrating a fourth example of coping information 130. In the coping information 130, a situation in which the pump is operating is displayed. In the example in Fig. 12, "Administering △△ ml of medicine OX", "Remaining amount after administration is OO ml.", and "Please keep quiet during administration." are displayed, and a progressing status of the medicine administration is graphically displayed, so that the progressing status can be grasped at a glance.

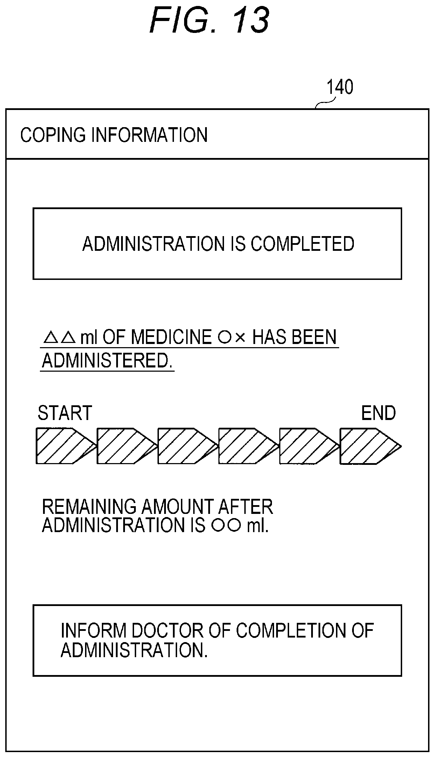

Fig. 13 is a diagram illustrating a fifth example of coping information 140. The coping information 140 displays completion of the medicine administration. In the example in Fig. 13, "△△ ml of medicine OX has been administered.", "Remaining amount after administration is OO ml.", and "Inform doctor of completion of administration." are displayed, and the completion of the administration of the medicine is graphically displayed.

As described above, the control unit 51 can control automatic administration of medicine in a medicine injection pump based on the pain evaluation result of the patient. As a result, for example, it is possible to automatically administer the medicine according to the situation of the pain of the patient, within a range of the prescription determined by the doctor in advance.

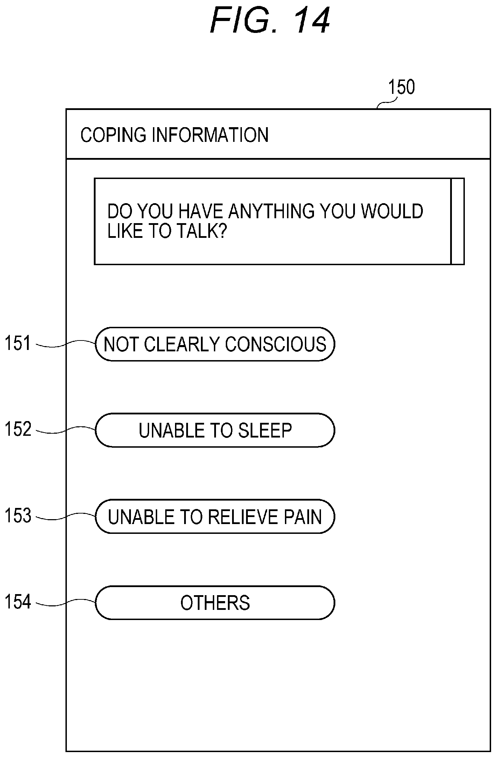

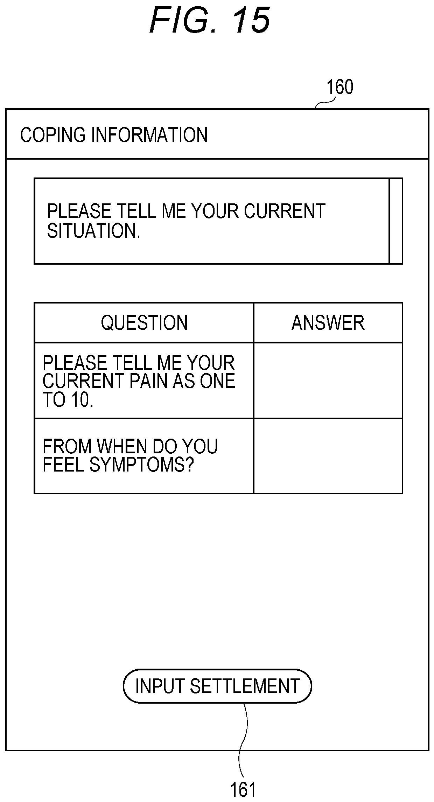

Fig. 14 is a diagram illustrating a sixth example of coping information 150. The coping information 150 includes information used to confirm the coping method. In the example in Fig. 14, by displaying "Do you have anything you would like to talk?", it is possible to confirm the symptom and the situation of the pain of the patient. Specifically, a "not clearly conscious" icon 151, an "unable to sleep" icon 152, an "unable to relieve pain" icon 153, an "others" icon 154, and the like are displayed, and the patient can select and operate an icon suitable for the symptom or the situation of the pain of the patient. For example, in a case where the patient feels not clearly conscious, the "not clearly conscious" icon 151 can be operated. As a result, coping information illustrated in Fig. 15 to be described later is displayed. Note that voice input or the like may be performed instead of the configuration for displaying and operating the icon.

Fig. 15 is a diagram illustrating a seventh example of coping information 160. The coping information 160 includes information used to confirm the coping method. In the example in Fig. 15, "Please tell me your current situation." is displayed, and a question used to determine the current situation of the patient in more detail is displayed. Although the question includes, for example, "Please tell me your current pain as one to 10.", "From when do you feel symptoms.", or the like, the questions are not limited to these. The patient inputs an answer to the question and operates an "input settlement" icon 161 so that the answer to the question is transmitted to the pain treatment assistance device 50. Note that the voice input may be used.

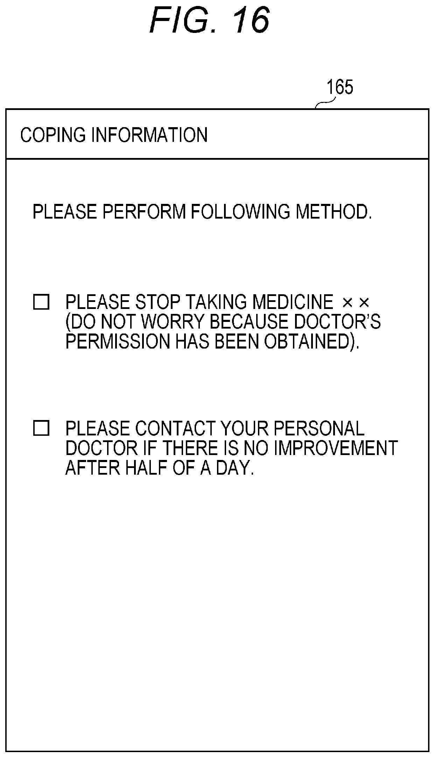

Fig. 16 is a diagram illustrating an eighth example of coping information 165. The coping information 165 includes prescription of medicine and recommendation of contacting the doctor. In the example in Fig. 16, "Please perform following method.", "Please stop taking medicine XX. (do not worry because doctor's permission has been obtained.)", and "Please contact your personal doctor if there is no improvement after half of a day." are displayed. As a result, the patient can stop taking the medicine XX and wait and see for a while. If the symptom of "not clearly conscious" is improved, it can be determined that there is no need to contact the personal doctor. Furthermore, in a case where the patient is not clearly conscious even if the patient stops taking the medicine XX, it is possible to contact the personal doctor and can have examination by the doctor or the like. Note that voice may be output instead of displaying characters. The coping information as illustrated in Fig. 16 can be stored in the storage unit 59 as a table associated with a combination of a plurality of answers in advance. For example, pieces of coping information S1, S2, S3,... are associated with combinations W1, W2, W3,... of the answers. The control unit 51 can select the combination of the answers illustrated in Fig. 15 from the table and specify the coping information associated with the selected combination of the answers.

As described above, the control unit 51 may receive a situation of the patient as a result of performing the coping information and recommend examination by the doctor according to the received situation.

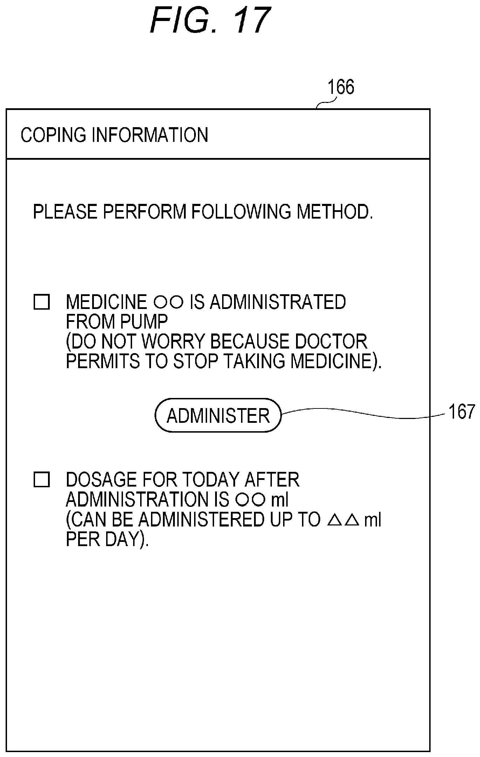

Fig. 17 is a diagram illustrating a ninth example of coping information 166. The ninth example indicates coping information in a case where the patient operates the "unable to relieve pain" icon 153 in the sixth example in Fig. 14. For example, as illustrated in Fig. 17, "Medicine OO is administrated from pump (do not worry because doctor permits to stop taking medicine)" is displayed. When the patient operates an "administer" icon 167, it is possible to administer the medicine. Furthermore, "Dosage for today after administration is OO ml. (can be administered up to △△ ml per day)" may be displayed. As a result, in a case where the pain is not relieved, the medication set in the pump can be administered to the patient. Note that voice may be output instead of displaying characters.

As described above, the control unit 51 can receive consultation from the patient and output the coping information according to the received consultation to the patient terminal device 10.

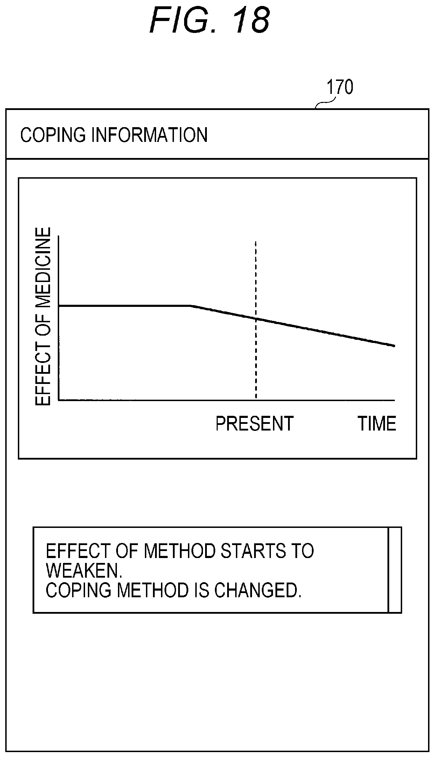

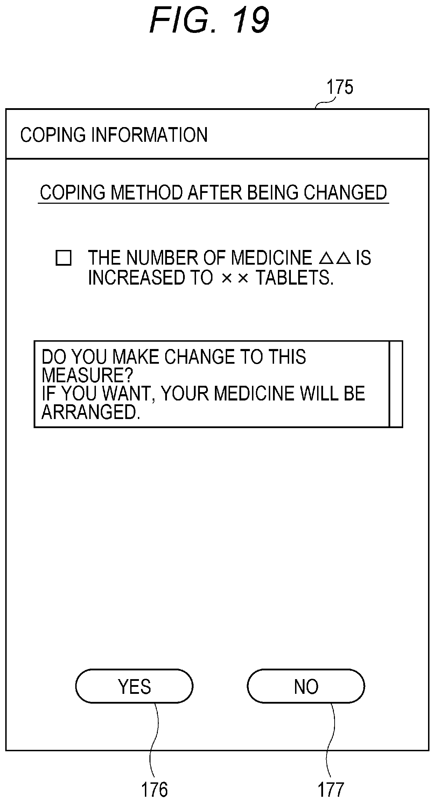

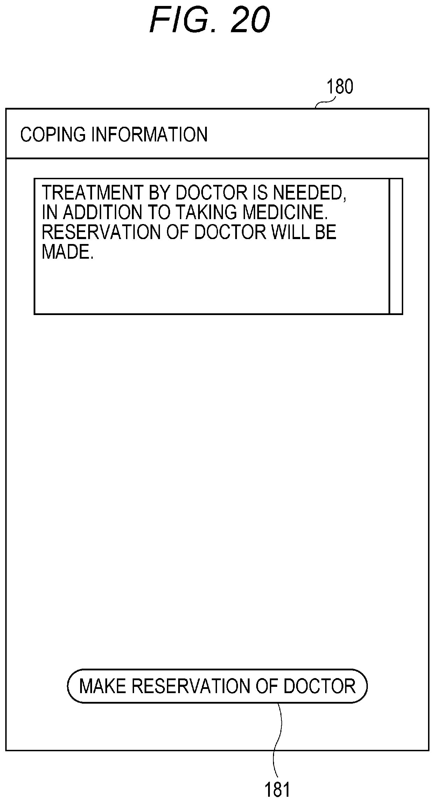

Fig. 18 is a diagram illustrating a tenth example of coping information 170. In the coping information 170, a progress pattern of an effect of medicine is displayed. When the effect of the medicine is set to y and the pain is set to x, a relational expression between the effect of the medicine y and the pain x can be expressed, for example, as y = K/x. The reference K is a constant. Note that the relational expression is not limited to this. In the example in Fig. 18, the progress pattern of the effect of the medicine is displayed as a line graph, and a transition of the effect of the medicine of the patient from the past to the future can be seen. A question based on the progress pattern of the effect of the medicine is displayed for the patient as if the doctor asks a question (interview) for the patient. The question is, for example, "The effect of the method starts to weaken. The coping method is changed". As a result, the patient can obtain an environment similar to an environment as if the patient is examined by the doctor. Next, the pain treatment assistance device 50 can display coping information as illustrated in Fig. 19 or Fig. 20 to be described later on the patient terminal device 10.

Fig. 19 is a diagram illustrating an eleventh example of coping information 175. The coping information 175 includes a coping method after being changed. In the example in Fig. 19, "The number of medicine △△ is increased to XX tablets." is displayed, and "Do you make change to this measure? If you want, your medicine will be arranged." is displayed. If the patient operates a "Yes" icon 176, the medicine is arranged. The medicine arrangement may be any one of the following. For example, (1) medicine based on a prescription is delivered to the home of the patient, (2) medicine based on the prescription is prepared at a pharmacy designated by the patient, (3) the prescription is transmitted to the patient terminal device 10, or the like. Furthermore, regarding the change of the medicine, the doctor may determine a change option at the time of interview. Furthermore, permission of the doctor may be obtained in a case where the medicine is arranged. In a case where the patient operates a "No" icon 177, the medicine is not arranged. The coping method as illustrated in Fig. 19 can be stored in the storage unit 59 in advance as a table associated with the plurality of progress patterns of the effect of the medicine. For example, coping methods T1, T2, T3,... are associated with progress patterns of the effect of the medicine M1, M2, M3,.... The control unit 51 can select a progress pattern of the effect of the medicine closest to the progress pattern of the effect of the medicine of the patient as illustrated in Fig. 18 from the table and specify a coping method associated with the selected progress pattern of the effect of the medicine.

Fig. 20 is a diagram illustrating a twelfth example of coping information 180. The coping information 180 includes recommendation of a treatment by a doctor and reservation of the doctor. In the example in Fig. 20, "Treatment by doctor is needed, in addition to taking medicine. Reservation of doctor will be made." is displayed. In a case of determining that the situation of the patient is not improved only by taking the medicine, based on the progress pattern of the effect of the medicine (pain progress pattern) of the patient, the pain treatment assistance device 50 can recommend intervention by the doctor, that is, the examination by the doctor. When the patient operates a "make reservation of doctor" icon 181, display of information and processing necessary for the reservation of the doctor are performed.

As described above, the control unit 51 can output the change in the coping information to the patient terminal device 10 based on the effect of the medicine of the patient. Furthermore, the control unit 51 can receive the arrangement of the medicine or contacting the doctor, together with the change in the coping information.

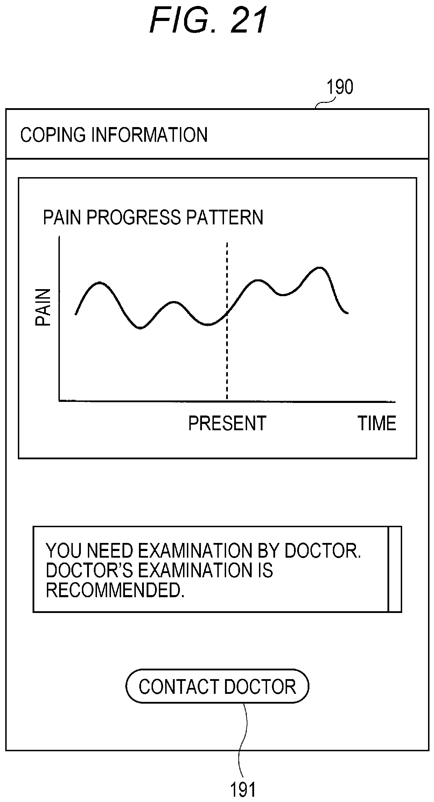

Fig. 21 is a diagram illustrating a thirteenth example of coping information 190. In the coping information 190, the pain progress pattern of the patient is displayed. In the example in Fig. 21, in the pain progress pattern, a situation where the pain usually continues and the pain becomes stronger or weaker with time repeatedly occurs. In this way, in a case where the pain progress pattern indicates a complicated variation, for example, "You need examination by doctor. Doctor's examination is recommended." is displayed. When the patient operates a "contact doctor" icon 191, processing necessary for contacting the doctor is executed.

As described above, the control unit 51 may recommend the examination by the doctor based on the pain evaluation result.

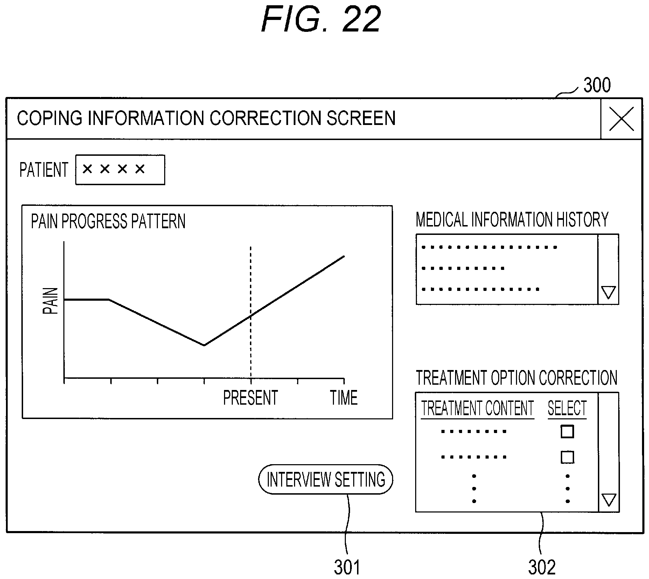

Fig. 22 is a diagram illustrating an example of a coping information correction screen 300. The coping information correction screen 300 is displayed on the doctor terminal device 40 in response to a request from the doctor terminal device 40. The doctor can display the coping information correction screen 300 on the doctor terminal device 40 and periodically observe the symptom and the pain progress of the patient. In the example in Fig. 22, a progress of a patient XXXX is displayed. The coping information correction screen 300 displays the pain progress pattern of the patient, a medical information history, a treatment option correction 302, or the like. The treatment option correction 302 includes coping information (treatment content) generated based on the pain evaluation result of the patient. The doctor can determine whether or not it is necessary to correct a treatment policy that is currently performed on the patient, by observing the pain progress pattern of the patient and the medical information history. In a case where the doctor determines that it is necessary to correct the treatment policy, it is possible to select a required treatment from the treatment content displayed in the treatment option correction 302. The selection of the treatment includes a change of the treatment, addition of the treatment, deletion of the treatment, or the like. Furthermore, in a case where the doctor determines that the interview with the patient is necessary, by operating an "interview setting" icon 301, processing necessary for interview setting is executed.

As described above, the control unit 51 can receive an operation for correcting the coping information generated based on the pain evaluation result of the patient from the doctor terminal device 40.

As described above, according to the present embodiment, it is possible to grasp the pain and the symptoms felt daily by the patient in real time and in a timely manner, to lead to relieve the pain, and to improve the QOL of the patient. Furthermore, the pain and the symptoms from the past to the future of the patient can be grasped, and occurrence and an increase of the pain can be suppressed in advance.

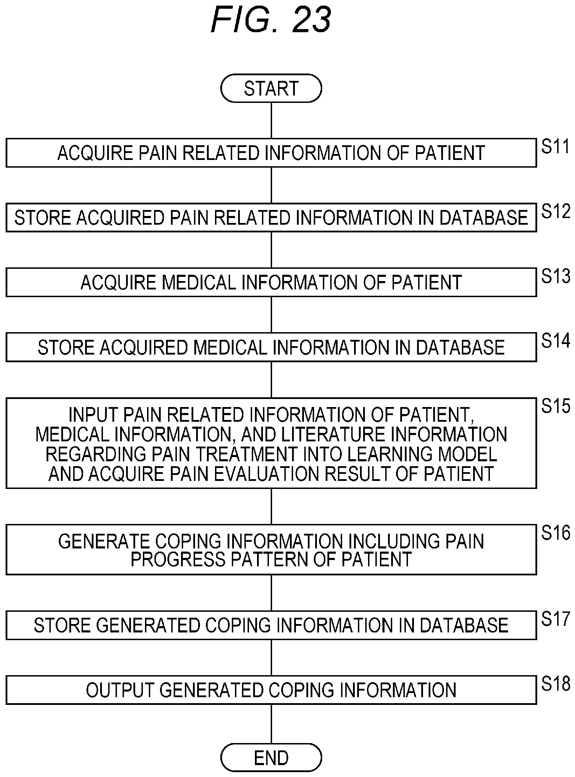

Fig. 23 is a diagram illustrating an example of a processing procedure by the pain treatment assistance device 50. Hereinafter, for convenience, the control unit 51 will be described as a subject of the processing. The control unit 51 acquires the pain related information of the patient (S11) and stores the acquired pain related information in the pain related information DB 54 (database) (S12). The pain related information can be repeatedly acquired at a required timing for each patient. For example, it can be acquired once or a plurality of times within a period from the time of the previous hospital visit to the time of the next hospital visit.

The control unit 51 acquires the medical information of the patient (S13) and stores the acquired medical information in the medical information DB 55 (database) (S14). The medical information can be acquired not only at the time of examination but also when the doctor determines that the medical information is needed.

The control unit 51 inputs the pain related information of the patient, the medical information, and the literature information regarding the pain treatment into the learning model 61 and acquires the pain evaluation result of the patient, output by the learning model 61 (S15). The control unit 51 generates the coping information including the pain progress pattern of the patient, based on the pain evaluation result of the patient acquired from the learning model 61 (S16). In this case, the coping information may be generated based on the pain related information of the patient, the medical information, and the literature information.

The control unit 51 stores the generated coping information in the coping information DB 56 (database) (S17), outputs the generated coping information (S18), and ends the processing.

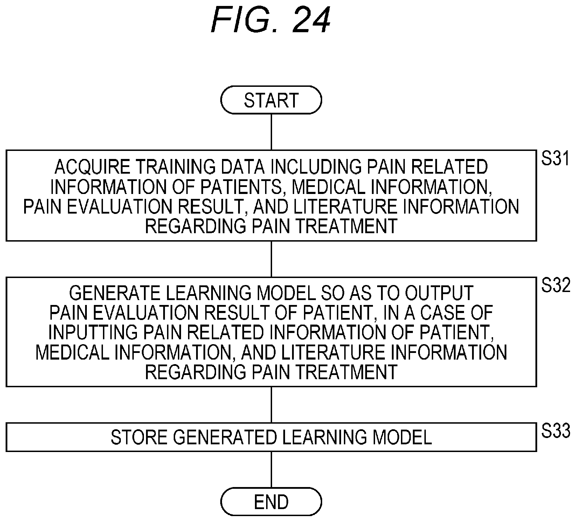

Fig. 24 is a diagram illustrating an example of a procedure for generating the learning model 61. The control unit 51 acquires the training data including the pain related information regarding the pain of the plurality of patients, the medical information, the pain evaluation result, and the literature information regarding the pain treatment (S31). The control unit 51 generates the learning model 61 so as to output the pain evaluation result of each patient, in a case of inputting the pain related information of each patient, the medical information, and the literature information regarding the pain treatment, based on the acquired training data (S32). The control unit 51 stores the generated learning model 61 in the storage unit 59 (S33) and ends the processing.

### Reference Signs List

- 1: communication network
- 10: patient terminal device
- 20: device
- 30: assistant terminal device
- 40: doctor terminal device
- 50: pain treatment assistance device
- 51: control unit
- 52: communication unit
- 53: memory
- 54: pain related information DB
- 55: medical information DB
- 56: coping information DB
- 57: literature information DB
- 58: coping information generation unit
- 59: storage unit
- 60: computer program
- 61: learning model

## Claims

1. A computer program for causing a computer to execute processing comprising:
acquiring pain related information regarding pain of a patient;
in a case where the pain related information is input, inputting the acquired pain related information into a learning model that outputs a pain evaluation result and acquiring the pain evaluation result of the patient;
generating coping information for coping with the pain based on the acquired pain evaluation result; and
outputting the generated coping information.

2. The computer program according to claim 1, wherein
the pain related information includes
at least one of an evaluation index used to evaluate pain, vital data, medication information, and a physical condition.

3. The computer program according to claim 1, for causing the computer to execute processing further comprising:
acquiring medical information regarding medical care of the patient; and
in a case where the medical information is further input, inputting the acquired medical information into the learning model that outputs the pain evaluation result and acquiring the pain evaluation result of the patient.

4. The computer program according to claim 3, wherein
the medical information includes
at least one of diagnosis information, examination information, treatment information, prescription information, and disease prognosis prediction information.

5. The computer program according to claim 1, for causing the computer to execute processing further comprising:
acquiring literature information regarding a pain treatment; and
in a case where the literature information regarding the pain treatment is further input, inputting the acquired literature information into the learning model that outputs the pain evaluation result and acquiring the pain evaluation result of the patient.

6. The computer program according to any one of claims 1 to 5, wherein
the pain evaluation result includes
a pain progress of the patient from the past to the future.

7. The computer program according to any one of claims 1 to 5, for causing the computer to execute processing further comprising:
receiving a situation of the patient as a result of performing the coping information; and
recommending examination by a doctor according to the received situation.

8. The computer program according to any one of claims 1 to 5, for causing the computer to execute processing further comprising:
receiving a situation of the pain of the patient as a result of performing a pain treatment based on the coping information;
correcting the pain evaluation result based on the received situation of the pain; and
relearning the learning model using the corrected pain evaluation result.

9. The computer program according to any one of claims 1 to 5, for causing the computer to execute processing further comprising:
receiving an operation for correcting the coping information generated based on the pain evaluation result of the patient from a doctor terminal device.

10. The computer program according to any one of claims 1 to 5, for causing the computer to execute processing further comprising:
outputting a question generated based on the pain evaluation result of the patient to a patient terminal device; and
outputting coping information to the patient terminal device according to an answer of the patient to the question.

11. The computer program according to any one of claims 1 to 5, for causing the computer to execute processing further comprising:
receiving consultation from the patient; and
outputting the coping information to a patient terminal device according to the received consultation.

12. The computer program according to any one of claims 1 to 5, for causing the computer to execute processing further comprising:
receiving arrangement of medicine or contacting a doctor, together with the coping information.

13. The computer program according to any one of claims 1 to 5, for causing the computer to execute processing further comprising:
controlling automatic administration of medicine in a medicine injection pump based on the pain evaluation result of the patient.

14. An information processing device comprising:
a first acquisition unit that acquires pain related information regarding pain of a patient;
a second acquisition unit that inputs the acquired pain related information into a learning model that outputs a pain evaluation result, in a case of inputting the pain related information and acquires the pain evaluation result of the patient;
a generation unit that generates coping information for coping with pain based on the acquired pain evaluation result; and
an output unit that outputs the generated coping information.

15. An information processing method comprising:
acquiring pain related information regarding pain of a patient;
inputting the acquired pain related information into a learning model that outputs a pain evaluation result, in a case where the pain related information is input, and acquiring the pain evaluation result of the patient;
generating coping information for coping with the pain based on the acquired pain evaluation result; and
outputting the generated coping information.

16. A learning model generation method comprising:
acquiring first training data including pain related information regarding pain of a plurality of patients and pain evaluation results of the plurality of patients; and
generating a learning model so as to output the pain evaluation result in a case where the pain related information is input, based on the acquired first training data.

17. The learning model generation method according to claim 16, further comprising:
acquiring second training data further including medical information of the plurality of patients, literature information regarding a pain treatment, and the pain evaluation results of the plurality of patients; and
generating the learning model so as to output the pain evaluation result in a case where the medical information and the literature information regarding the pain treatment are input, based on the acquired second training data.
